# EUROPEAN PATENT APPLICATION

(11) **EP 0 747 029 A1**
(43) Date of publication of application: **11.12.1996**
(21) Application number: 95108884.8
(22) Date of filing: 09.06.1995
(51) Int. Cl.: A61F 13/15, A61F 13/52

(54) **Absorbent article comprising barrier cuffs and an anti-wicking topsheet region located inboard from the barrier cuffs**

(71) Applicant: THE PROCTER & GAMBLE COMPANY, Cincinnati, Ohio 45202 (US)
(72) Inventor: Vinas, Bernard, D-65835 Liederbach (DE)
(74) Representative: Bottema, Johan Jan

(57) **Abstract**

The invention relates to an absorbent article comprising barrier cuffs along its longitudinal sides. The article comprises a topsheet having a central region and marginal regions. The marginal regions extend outwardly below the cuffs. The marginal regions are more hydrophobic than the central region throughout their thickness to prevent wicking of liquids below the cuffs. The topsheet may be comprised of a hydrophilic material, the marginal areas being impregnated with a hydrophobic agent. alternatively, the topsheet may be comprised of a hydrophobic material, the central region being treated with a surfactant.

## Description

### Field of the Invention

The invention relates to an absorbent article comprising:
- a liquid-impervious backsheet,
- a liquid-pervious topsheet of fibrous material,
- an absorbent core comprising two longitudinal edges, two end areas and a crotch area located between the end areas, wherein the core is interposed between the topsheet and the backsheet, the topsheet and the backsheet extending laterally outwardly beyond the longitudinal edges of the core, and
- a barrier cuff located outwardly along each longitudinal edge of the core, each barrier cuff comprising a proximal edge connected to the topsheet, the topsheet extending laterally outwardly from the proximal edges of the barrier cuffs.

### Background of the Invention

US-A-4,695,278 (Lawson) discloses an absorbent article having a topsheet and a backsheet which are coterminous. Elasticised upstanding barrier cuffs are connected along longitudinal side margins of the absorbent core to prevent lateral migration of liquids. The barrier cuffs may be made of non-woven material which is made liquid-impermeable by treatment of the cuff or by securing a separate material to the cuff. In this construction leakage of liquids via wicking through the topsheet below the cuffs may take place.

US-A-4,795,545 (Dragoo) discloses an absorbent article having a topsheet and a backsheet which are substantially non-coterminous. Elasticised barrier cuffs are provided, and are connected to the backsheet along their proximal edge. The longitudinal edges of the topsheet are located inboard from the proximal edges of the barrier cuffs. In this way a barrier against leakage below the barrier cuffs is formed.

US-A-4,718,898 discloses a liquid-impermeable waste barrier, provided on the topsheet of a diaper, to prevent leakage along the perimeter. The barrier is formed by coating a part of the topsheet with a hot-melt adhesive, which forms a liquid impermeable film on the topsheet.

It is an object of the present invention to provide an absorbent article comprising barrier cuffs, wherein leakage of liquids below the barrier cuffs is prevented.

It is another object of the present invention to provide an absorbent article, which is of relatively simple construction.

It is a further object of the invention to provide an absorbent article which comprises a layer of fibrous material in its side marginal regions, which is of a cost-effective construction.

### Summary of the Invention

An absorbent article in accordance with the present invention comprises a topsheet having:
- at least in the crotch area a marginal region that is adjacent to the proximal edges and that is located at least partly inboard from the proximal edges, and
- a hydrophilic central region comprised between the marginal regions, wherein the topsheet material is treated in the central region with a wetting agent which coats the fibers of the topsheet and renders the central region more hydrophilic than the marginal regions, the marginal regions being sufficiently hydrophobic substantially throughout the entire thickness of the topsheet, to prevent wicking of liquids via the topsheet underneath the barrier cuffs.

Alternatively, the topsheet of an absorbent article according to the present invention comprises:
- at least in the crotch area a marginal region that is adjacent to the proximal edges and that is located at least partly inboard from the proximal edges, and
- a hydrophilic central region comprised between the marginal regions, wherein the marginal regions are impregnated with a hydrophobic agent which coats the fibers of the topsheet substantially throughout the entire thickness of the topsheet and renders the marginal regions more hydrophobic than the central region to prevent wicking of liquids via the topsheet underneath the barrier cuffs.

The topsheet of the absorbent article according to the invention extends towards the periphery of the absorbent article, and provides a soft layer for contacting the skin in these regions. Hence the peripheral regions of the backsheet need not be separately covered by an individual strip of soft, fibrous material. In this way, a simplification of the method of manufacturing an absorbent article is achieved.

Furthermore, as the topsheet extends across the peripheral areas of the absorbent article, there may during production of the absorbent article be elastic elements placed on the topsheet in these regions. Prior to combining the topsheet with the backsheet, leg elastics or elastic waist panels may be placed onto the peripheral areas of the topsheet in their relaxed state or in a pre-stretched manner. As the topsheet is air-permeable, it is advantageous to place the elastic features onto the topsheet, as they can be maintained on the topsheet -when necessary in a pre-stretched state- by suction from a vacuum device below the topsheet.

As the topsheet extends outwardly below the barrier cuffs, the backsheet material in the periphery of the article need not be covered by the material of the barrier cuffs. The material from which the barrier cuffs are made typically comprises a non-woven material or a coated non-woven material, and will in general be a more costly material than the topsheet-material. Using relatively narrow strips of barrier cuff material, is desirable from a cost perspective.

The present invention furthermore allows the proximal edges of the cuffs to be bonded to the topsheet in discrete points without the necessity of the attachment of the cuffs to the topsheet forming a continuous and liquid-tight seal along the whole length of the proximal edges.

If the topsheet material is inherently hydrophilic, lateral wicking of liquids is prevented by coating the fibers in the side marginal regions, inwardly alongside of the barrier cuffs, with a hydrophobic agent. Contrary to the known application of hot melt adhesive to a fibrous sheet, the hydrophobic agent coats the surface of the fibers substantially throughout the entire thickness of the topsheet. Hereby the lateral liquid migration through the side marginal regions of the topsheet is prevented. In this case, the topsheet may for instance be formed of cellulose fibers, the marginal areas being impregnated with a silicone-containing solution.

If the topsheet material is inherently hydrophobic, no substantial lateral wicking of liquids through the topsheet material will take place. To allow entry of liquids into the absorbent article into the core in a vertical direction, the central region of the topsheet is treated with a hydrophilic, or wetting agent. During treatment of the central region of the topsheet, care is taken that the side marginal regions are shielded from the wetting agent. In this case, the topsheet may be formed by polypropylene fibers, the central region being treated with a surfactant.

The term "hydrophilic" is intended to describe topsheet materials which have a contact angle with water of less than 90°. A material is considered to be hydrophilic if water or aqueous body fluid readily spreads on or over the surface of a fiber of said material. The degree of hydrophilicity can be quantified by referencing the advancing contact angle between a drop of water and the horizontal surface of a fiber.

The contact angle concept is well-known. In general, advancing contact angle can be defined for purposes of this invention as the angle between the surface of the fiber and the vector which is tangent to a water drop at the point of its contact with the fiber surface, i.e. at the point of the interline between the drop and the surface. Contact angle is generally measured in the liquid phase as the interline advances over the fiber surface and, for purposes of this invention, is determined at a temperature of 20°C and relative humidity of 65 %. Determination of advancing contact angle between water and fiber can be made in the general manner described in Damson, A. W., Physical Chemistry of Surfaces, Wiley-Interscience, 3rd Ed., 1976, or in Daugherty, T.H., "Dynamic Wetting of Single Pulp Fibers, MS Thesis, University of Washington, 1981, both of which publications are incorporated herein by reference. Preferably the hydrophilic fibers of the topsheet will have an advancing contact angle with water of 90° or less, more preferably will between about 0° and 60°. Examples of hydrophilic materials used in topsheets are cellulose fibers or nylon fibers.

The term "hydrophobic" is intended to describe topsheet materials which have a contact angle with water of more than 90°. Examples of hydrophobic materials used in topsheets are polyethylene fibers, spun bonded polypropylene fibers or PET fibers. Topsheets made of carded fibers may be hydrophobic because during the carding process, hydrophobic anti-static substances are used.

The term "hydrophobic agent" is intended to indicate a substance which is able of coating the fibrous topsheet material and of imparting to those fibers a wetting angle of 90° or more.

In the definition of present invention, the difference in hydrophilicity between the central region of the topsheet and the side marginal regions thereof, may be characterised by a difference in contact angles. Alternatively, the difference in hydrophilicity may be determined in a vertical wicking test, wherein the central region has a vertical wicking distance of a few cm and the side marginal regions have a vertical wicking distance of not more than a few mm.

### BRIEF DESCRIPTION OF THE DRAWINGS

The invention will be explained in detail with reference to the accompanying drawings. In the drawings:
Figure 1 shows a top plan view of an absorbent article according to the invention,

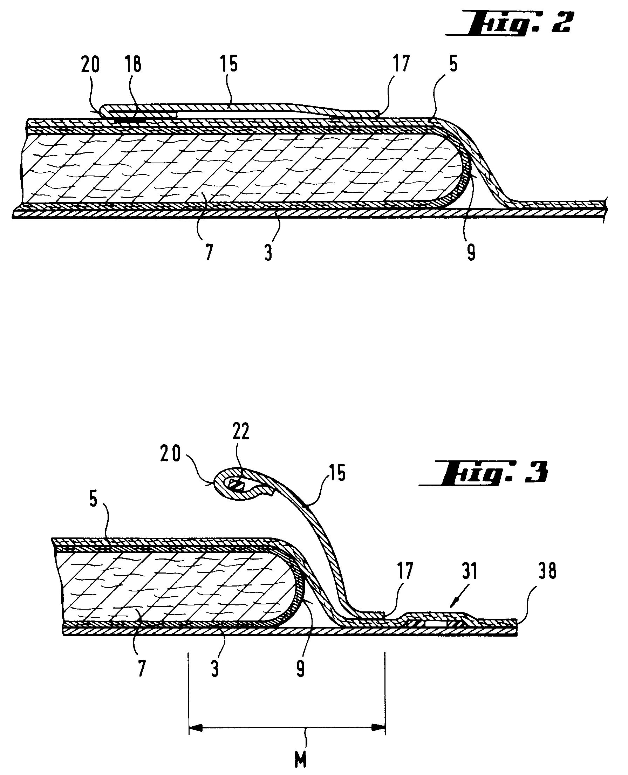

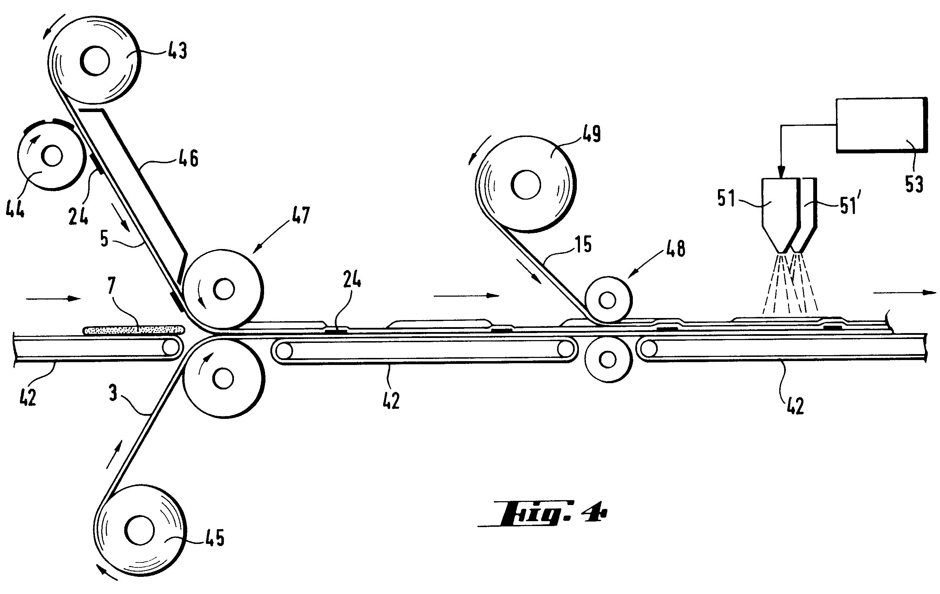

Figures 2 and 3 show cross-sectional views of the article of figure 1 along the lines 2-2 and 3-3 respectively,
Figure 4 shows a manufacturing line for forming an absorbent article according to the invention and
Figure 5 shows a cross-sectional view of an other embodiment of an absorbent article according to the invention.

### DETAILED DESCRIPTION OF THE INVENTION

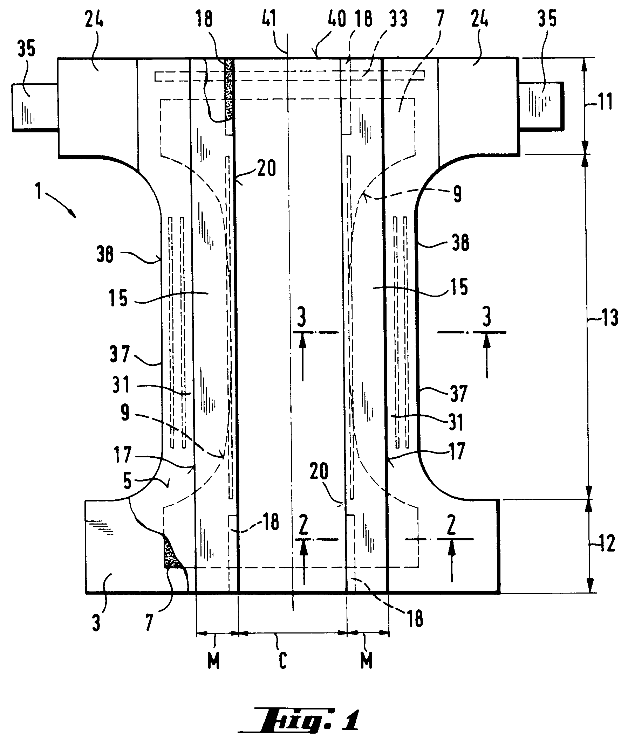

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A "unitary" absorbent article refers to absorbent articles which are formed of separate parts united together to form a co-ordinated entity so that they do not require separate manipulative parts like a separate holder and liner. A preferred embodiment of an absorbent article of the present invention is the unitary disposable absorbent article, diaper 1, shown in Figure 1. As used herein, the term "diaper" refers to an absorbent article generally worn by infants and incontinent persons that is worn about the lower torso of the wearer. It should be understood, however, that the present invention is also applicable to other absorbent articles such as incontinent briefs, incontinent undergarments, diaper holders and liners, feminine hygiene garments, and the like.

Figure 1 is a plan view of the diaper 1 of the present invention in its flat-out, state (i.e., with elastic induced contraction pulled out) with portions of the structure being cut-away to more clearly show the construction of the diaper 1 and with the portion of the diaper 1 which faces or contacts the wearer, the inner surface, oriented towards the viewer. As shown in Figure 1, the diaper 1 preferably comprises a liquid pervious topsheet 5; a liquid impervious backsheet 3 joined with the topsheet 5; an absorbent core 7 positioned between the topsheet 5 and the backsheet 3; elasticised side panels; an elastic waist feature 33; a fastening system generally multiply designated as 35; and barrier cuffs 15.

The diaper 1 is shown in Figure 1 to have a first end area 11, a second end area 12, a crotch area 13 positioned between the first end area 11 and the second end area 13, and a periphery 37 which is defined by the outer edges of the diaper 1. The longitudinal edges are designated 38 and the end edges are designated 40.

Figure 1 shows a preferred embodiment of the diaper 1 in which the topsheet 5 and the backsheet 3 have length and width dimensions generally larger than those of the absorbent core 7. The topsheet 5 and the backsheet 3 extend beyond the longitudinal edges 9 of the absorbent core 7 to thereby form the periphery 37 of the diaper 1. While the topsheet 5, the backsheet 3, and the absorbent core 7 may be assembled in a variety of well known configurations, preferred diaper configurations are described generally in U.S. Patent 3,860,003 entitled "Contractible Side Portions for Disposable Diaper" which issued to Kenneth B. Buell on January 14, 1975; and U.S. Patent Application Serial No. 07/715,152, allowed, "Absorbent Article With Dynamic Elastic Waist Feature Having A Predisposed Resilient Flexural Hinge", Kenneth B. Buell et al. filed June 13, 1991; each of which is incorporated herein by reference.

The absorbent core 7 may be any absorbent means which is generally compressible, conformable, non-irritating to the wearer's skin, and capable of absorbing and retaining liquids such as urine and other certain body exudates. As shown in Figure 1, the absorbent core 7 has a garment surface, a body surface, side edges, and waist edges. The absorbent core 7 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding; meltblown polymers including conform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials. The configuration and construction of the absorbent core may also be varied (e.g., the absorbent core may have varying caliper zones, a hydrophilic gradient, a superabsorbent gradient, or lower average density and lower average basis weight acquisition zones; or may comprise one or more layers or structures). The total absorbent capacity of the absorbent core 7 should, however, be compatible with the design loading and the intended use of the diaper 1. Further, the size and absorbent capacity of the absorbent core 7 may be varied to accommodate wearers ranging from infants through adults. Exemplary absorbent structures for use as the absorbent core 7 are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989. Each of these patents are incorporated herein by reference.

The backsheet 3 is positioned adjacent the garment surface of the absorbent core 7 and is preferably joined thereto by attachment means (not shown) such as those well known in the art. For example, the backsheet 3 may be secured to the absorbent core 7 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Each of these patents are incorporated herein by reference. Alternatively, the attachment means may comprise heat bonds, pressure bonds, ultrasonic bonds, dynamic mechanical bonds, or any other suitable attachment means or combinations of these attachment means as are known in the art.

The backsheet 3 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet 3 prevents the exudates absorbed and contained in the absorbent core 7 from wetting articles which contact the diaper 1 such as bedsheets and undergarments. The backsheet 3 may thus comprise a woven or nonwoven material, polymeric films such as thermoplastic films of polyethylene or polypropylene, or composite materials such as a film-coated nonwoven material. Preferably, the backsheet is a thermoplastic film having a thickness of from about 0.012 mm (0.5 mil) to about 0.051 mm (2.0 mils). Particularly preferred materials for the backsheet include RR8220 blown films and RR5475 cast films as manufactured by Tredegar Industries, Inc. of Terre Haute, IN. The backsheet 3 is preferably embossed and/or matte finished to provide a more clothlike appearance. Further, the backsheet 3 may permit vapors to escape from the absorbent core 7 (i.e., breathable) while still preventing exudates from passing through the backsheet 26.

The topsheet 5 is positioned adjacent the body surface of the absorbent core 7 and is preferably joined thereto and to the backsheet 3 by attachment means (not shown) such as those well known in the art. Suitable attachment means are described with respect to joining the backsheet 3 to the absorbent core 7. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. In a preferred embodiment of the present invention, the topsheet 5 and the backsheet 3 are joined directly to each other in the diaper periphery 37 and are indirectly joined together by directly joining them to the absorbent core 7 by the attachment means (not shown).

The topsheet 5 is a fibrous material which is compliant, soft feeling, and non-irritating to the wearer's skin. Further, the topsheet 5 is liquid pervious permitting liquids (e.g., urine) to readily penetrate through its thickness. A suitable topsheet may be manufactured from a wide range of materials, such as woven or nonwoven webs of natural fibers (e.g., wood or cotton fibers), synthetic fibers (e.g., polyester or polypropylene fibers), or a combination of natural and synthetic fibers. Preferably, the topsheet 5 is made of a hydrophobic material to isolate the wearer's skin from liquids contained in the absorbent core 7. There are a number of manufacturing techniques which may be used to manufacture the topsheet 5. For example, the topsheet 5 may be a nonwoven web of fibers spunbonded, carded, wet-laid, meltblown, hydroentangled, combinations of the above, or the like. A preferred topsheet is carded and thermally bonded by means well known to those skilled in the fabrics art. A preferred topsheet comprises a web of staple length polypropylene fibers such as is manufactured by Veratec, Inc., a Division of International Paper Company, of Walpole, Massachusetts under the designation P-8.

In the marginal regions M,M' of the topsheet 5, two barrier cuffs 15 are connected to the topsheet with their proximal edges 17. Each barrier cuff 15 comprises a free edge 20 which is elasticated by elastic spacing means 22. The spacing means 22 contract and gather the free edge 20 which thereby is lifted and is spaced-away from the topsheet 5. The barrier cuffs 15 may be formed from the same material as the topsheet 5, the backsheet 3 or may be a laminate of a non-woven web and a liquid-impermeable film. The topsheet 5 extends below the proximal edges 17 of the cuffs 15 in a direction of the periphery 37 of the diaper 1. Inboard from the cuffs 15, in marginal regions M,M' the topsheet 5 is hydrophobic throughout its thickness. The hydrophobic properties of the topsheet 5 may be derived from the hydrophobic nature of the fiber material in the marginal regions M, M', or may be provided by applying a hydrophobic coating such as a silicone-containing emulsion to the fibers of the topsheet 5 in the marginal regions.

When the topsheet 5 is made of hydrophobic fibers, such as for instance polypropylene fibers, the central region C of the topsheet is coated with a surfactant. The coating may be carried out by spraying or printing the central region with surfactant or by dipping this region into the surfactant. Suitable surfactants include non-ionic surfactants such as Brij 76 manufactured by ICI Americas, Inc. of Wilmington, Delaware and the various materials sold under the Pegosperse trademark by Glyco Chemical, Inc. of Greenwich, Connecticut. Anionic surfactants can be also used. The contact angle of water with the fibers in the side marginal regions M,M' will in this case preferably be greater than 90°, the contact angle of water with the fibers the central area being smaller than 90°. In a vertical wicking test, the wicking through the topsheet in the central region C is of the order of magnitude of a few cm. The wicking of water through the marginal regions M,M' is preferably neglible in a vertical wicking test.

Alternatively, the topsheet 5 may be formed of cellulosic fibers or other hydrophilic fibers, the side marginal regions M,M' being impregnated with a hydrophobic agent. In this case the same difference in hydrophilicity is achieved as above, and liquids are prevented from wicking through the topsheet 5 below the cuffs 15. The side marginal areas M,M' may extend below the cuffs 15 and may terminate at the diaper periphery 37.

Both the cuffs 15 and the marginal areas M,M' need not extend along the full length of the diaper 1, but may be confined to the crotch area 13 only. In the end areas, the cuffs 15 are secured to the topsheet with their free edges 20 by adhesive securement means 18. This attachment prevents the cuffs 15 from being flipped outwardly upon use. However, in alternative embodiments, the free edge 20 may be attached for instance in the rear area 11, outboard from the proximal edge 17 of the cuffs 15 to provide a larger spacing between opposite free edges 20 of the cuffs. Such outwardly flipped cuffs may be advantageous in the back waist region of an absorbent product which is to accommodate the buttocks of the wearer.

The marginal regions M,M' may extend from the proximal edges 17 of the cuffs 15 towards the longitudinal center line 41 of the diaper 1, and may be between 1 and 5 cm in width. The central region C of the topsheet 5 is between 5 and 10 cm in width.

Figure 4 schematically shows a production line for manufacturing an absorbent article according to the invention. On a transport means, such as a conveyor 42, absorbent cores 7 are passed between a combining nip 47. In the nip 47, the topsheet 5 and the backsheet 3, which are unrolled from supply rolls 43 and 45, are mutually connected. An applicator 44 applies elastomeric patches forming the elastic panels 24, to the topsheet 5. The elastic panels 24 may be maintained adhesively against the topsheet material, but can also, in view of the topsheet's permeability for air, be attached to the topsheet by means of a vacuum device 46 placed adjacent the topsheet 5. From a supply roll 49, the cuffs 15 are supplied. The cuffs 15 are bonded to the topsheet 5 by adhesive bonding, ultrasonic bonding or pressure bonding, along continuous or discontinuous bonds in a nip 48. When the cuffs 15 are bonded in discrete bond areas, liquids would in absence of the hydrophobic marginal regions M,M' be able to migrate through the topsheet 5 below the cuffs especially well in between these discrete bonding areas. In the absorbent article according to the present invention, wicking between the bond areas along the proximal edges 17 of the cuffs 15 is prevented by the hydrophobic marginal regions. Prior to combination of the cuffs 15 with the topsheet 5, elastic spacing elements 22 are attached to the cuffs (not shown in figure 4).

From a nozzle 51, a liquid coating material that is stored in a tank 53, is applied to the topsheet 5. If the topsheet 5 is made of a hydrophobic material, the nozzle 51 impregnates a central strip of the topsheet having a width that corresponds to the dimension of the central region C, with a hydrophilising agent. In case the topsheet 5 is made of a hydrophilic material, two nozzles 51, 51' may be provided, each nozzle 51, 51' impregnating a side marginal strip of the topsheet 5 with a hydrophobic agent. Instead of spray nozzles 51, 51', a gravure printing roll can be used in which the hyrophilising or hydrophobic agent is supplied from a reservoir contacting the rotating printing roll and is transferred from the surface of the printing roll onto the topsheet 5.

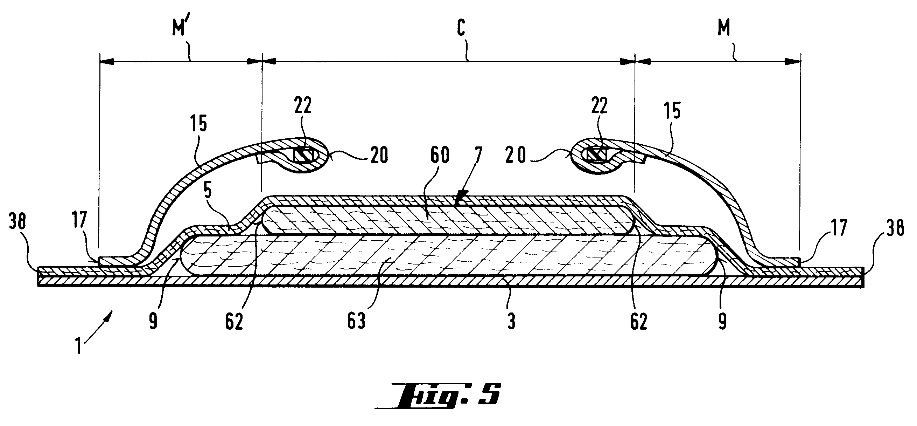

Figure 5 shows a cross sectional view of an embodiment of an absorbent article comprising an acquisition patch 60 overlying the central region C of the absorbent core 7. The acquisition patch 60 serves to rapidly acquire liquids and transport these liquids vertically into a lower storage layer 63 of the core 7. The acquisition patch may comprise chemically modified cellulose fibers as described in EP-A-0 427 317 or may comprise resilient thermally bonded synthetic fibers. The side marginal areas M,M' extend from the proximal edges 17 of the cuffs 15 to the edges 62 of the acquisition patch 60. In this embodiment the relatively hydrophobic side marginal areas M,M' prevent liquids from migrating from the sides of the acquisition layer 60, through the topsheet 5 towards the longitudinal edges 38 of the diaper 1. Furthermore, rewet of liquids from the core 7 back through the topsheet 5 to the skin of the wearer is prevented in the side marginal areas M,M' by the hydrophobic nature of the topsheet in these areas.

## Claims

1. Absorbent article (1) comprising:
- a liquid-impervious backsheet (3),
- a liquid-pervious topsheet (5) of fibrous material,
- an absorbent core (7) comprising two longitudinal edges (9), two end areas (11,12) and a crotch area (13) located between the end areas, wherein the core is interposed between the topsheet and the backsheet, the topsheet and the backsheet extending laterally outwardly beyond the longitudinal edges of the core, and
- a barrier cuff (15) located outwardly along each longitudinal edge (9) of the core, each barrier cuff comprising a proximal edge (17) connected to the topsheet, the topsheet extending laterally outwardly from the proximal edges of the barrier cuffs,
characterised in that
the topsheet comprises
- at least in the crotch area a marginal region (M,M') that is adjacent to the proximal edges (17) and that is located at least partly inboard from the proximal edges, and
- a hydrophilic central region (C) comprised between the marginal regions (M,M'), wherein the topsheet material is treated in the central region with a wetting agent which coats the fibers of the topsheet and renders the central region more hydrophilic than the marginal regions, the marginal regions being sufficiently hydrophobic substantially throughout the entire thickness of the topsheet, to prevent wicking of liquids via the topsheet underneath the barrier cuffs.

2. Absorbent article (1) comprising:
- a liquid-impervious backsheet (3),
- a liquid-pervious topsheet (5) of fibrous material,
- an absorbent core (7) comprising two longitudinal edges (9), two end areas (11,12) and a crotch area (13) located between the end areas, wherein the core (7) is interposed between the topsheet and the backsheet, the topsheet and the backsheet extending laterally outwardly beyond the longitudinal edges (9) of the core, and
- a barrier cuff (15) located outwardly along each longitudinal edge of the core, each barrier cuff (15) comprising a proximal edge (17) connected to the topsheet, the topsheet extending laterally outwardly from the proximal edges of the barrier cuffs,
characterised in that
the topsheet comprises
- at least in the crotch area (13) a marginal region (M,M') that is adjacent to the proximal edges (17) and that is located at least partly inboard from the proximal edges, and
- a hydrophilic central region (C) comprised between the marginal regions, wherein the marginal regions are impregnated with a hydrophobic agent which coats the fibers of the topsheet (5) substantially throughout the entire thickness of the topsheet and renders the marginal regions more hydrophobic than the central region to prevent wicking of liquids via the topsheet underneath the barrier cuffs (15).

3. absorbent article according to claim 2, wherein the topsheet is made of a hydrophilic material, the marginal regions being treated with a silicone-containing emulsion.

4. Absorbent article according to claim 1, wherein the topsheet is made of a hydrophobic material, the central region being treated with a surfactant.

5. Absorbent article according to claim 4, wherein the central region is sprayed with a surfactant.

6. Absorbent article according to any of the previous claims, wherein the difference in lateral wicking through the central region and lateral wicking through the side marginal regions of the topsheet, of a 0.1 saline solution in a vertical wicking test after 2 minutes, is at least 1 cm.

7. Absorbent article according to any of the previous claims, wherein core (7) comprises a storage layer (63) and an acquisition layer (60) located between the storage layer and the topsheet (5), the acquisition layer having longitudinal sides (62) which are located inboard from the longitudinal edges of the storage layer (63), the marginal region (M,M') extending outwardly from the longitudinal sides of the acquisition layer to at least the proximal edges of the barrier cuffs.

8. Absorbent article according to any of the previous claims, wherein the barrier cuffs (15) are bonded with their proximal edge (17) to the topsheet in discrete points.

9. Absorbent article according to claim 8, wherein the barrier cuffs are bonded to the topsheet by pressure bonding.
